# EUROPEAN PATENT SPECIFICATION

(11) **EP 2 264 060 B1**
(45) Date of publication and mention of the grant of the patent: **23.04.2014**
(21) Application number: 10189277.6
(22) Date of filing: 26.01.2007
(51) Int. Cl.: C07K 14/47, A61K 47/48, C12N 15/62, A61K 38/04

(54) **Compositions and methods for inhibiting viral adhesion**
Zusammensetzung und verfahren zur Bekämpfung von viralen Infektionen
Compositions et méthodes pour l'inhibition de l'adhésion virale.

(30) Priority: 26.01.2006 US 762796 P
(43) Date of publication of application: 22.12.2010
(62) Divisional of application: 07804693.5
(73) Proprietor: Recopharma AB, 14157 Huddinge (SE)
(72) Inventor: HOLGERSSON, Jan, 426 76 Vastra Frolunda (SE)
(74) Representative: Mintz Levin Cohn Ferris Glovsky and Popeo LLP

(56) References cited:
- WO-A-03/088996
- WO-A2-03/089450
- JOURNAL OF VIROLOGY., vol. 76, no. 17, 2002, pages 8834-8841, THE AMERICAN SOCIETY FOR MICROBIOLOGY. ISSN: 0022-538X

## Description

### FIELD OF THE INVENTION

The invention relates to generally to compositions and methods for treating or preventing viral infection and more particularly to compositions including fusion polypeptides comprising carbohydrate epitopes that mediate viral adhesion.

### BACKGROUND OF THE INVENTION

Specific cell surface attachment by virus particles is necessary for viral entry, replication and infection. Viruses use as receptors cell surface molecules involved in normal cellular functions. Such receptors are typically glycoproteins, and viral attachment can be both to the polypeptide or the glycan part of such glycoproteins. Viral receptors are not only important for attachment, but have been shown to trigger subsequent interactions with secondary receptors necessary for viral entry and replication. WO-A2-03089450 provides compounds for use in decreasing bacterial adhesion but provides no enabling disclosure for viruses.

### SUMMARY OF THE INVENTION

The inv**e**ntion is based in part on the discovery that carbohydrate epitopes that mediate viral attachment can be specifically expressed at high density and by different core saccharide chains on mucin-type protein backbones. The polypeptides are referred to herein as LAV fusion polypeptides. These recombinant, heavily glycosylated proteins carrying ample N-linked or O-linked glycans capped with carbohydrate determinants with known virus-binding activity can act as decoys, and as such specifically and sterically prevent virus infection in for example, the eye,
the respiratory or the gastrointestinal tracts. The fusion proteins have low toxicity and low risk of inducing viral resistance to the drugs.

In one aspect, the invention provides a fusion polypeptide for use in decreasing viral adhesion in a cell that includes a first polypeptide that carry one or more of the following carbohydrate epitopes Siaα3Galβ4GlcNAcβ, Siaα3Galβ3GlcNAcβ, operably linked to a second polypeptide. The first polypeptide is multivalent for these epitopes. The first polypeptide is a mucin polypeptide such as PSGL-1 or portion thereof. Preferably, the mucin polypeptide is the extracellular portion of PSGL-1. Alternatively, the first polypeptide is an alpha glycoprotein such as alpha 1-acid glycoprotein (i.e., orosomuciod or AGP) or portion thereof.

The second polypeptide comprises at least a region of an immunoglobulin polypeptide. For example, the second polypeptide comprises a region of a heavy chain immunoglobulin polypeptide. Alternatively, the second polypeptide comprises the FC region of an immunoglobulin heavy chain.

In another aspect, the invention provides compounds for use in a method of inhibiting (e.g., decreasing) viral attachment to a cell. Attachment is inhibited by contacting the virus with the AV fusion polypeptide. The invention also features methods of preventing or alleviating a symptom of an viral infection or a disorder associated with a viral infection in a subject by identifying a subject suffering from or at risk of developing a viral infection and administering to the subject a AV fusion polypeptide. The virus is for example, a Calicivirus or Influenza virus.

The subject is a mammal such as human, a primate, mouse, rat, dog, cat, cow, horse, pig. The subject is suffering from or at risk of developing a viral infection or a disorder associated with a viral infection. A subject suffering from or at risk of developing a viral infection or a disorder associated with a microbial infection is identified by methods known in the art

Also included in the invention are pharmaceutical compositions that include the AV fusion polypeptides.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

Other features and advantages of the invention will be apparent from the following detailed description, and from the claims.

### DETAILED DESCRIPTION OF THE INVENTION

The invention is based in part in the discovery that carbohydrate epitopes that mediate viral attachment can be specifically expressed at high density on glycoproteins, e.g., mucin-type and alpha glycoprotein protein backbones. This higher density of carbohydrate epitopes results in an increased valancy and affinity compared to monovalent oligosaccharides and wild-type, e.g. native non recombinantly expressed glycoproteins.

Table I lists examples of viruses attaching to host cells via binding to cell surface glycans.

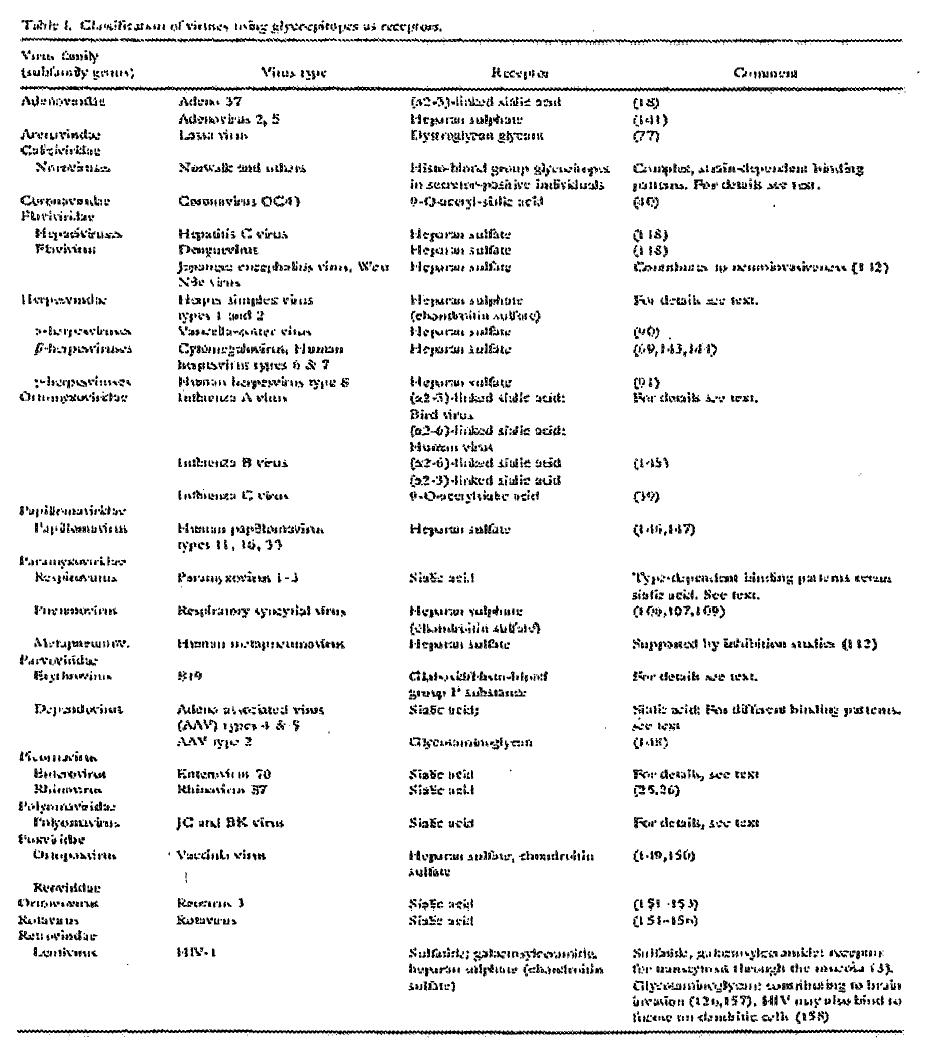

Adapted from Olofsson, S. et al. Annals of Medicine 2005, 37: 154-172, hereby incorporated by reference in its entirety

The carbohydrate epitopes, Siα3Galβ4GlcNAcβ, Siaα3Galβ3GlcNAcβ, are ligands for cell surface molecules. Many virus use a sialic acid receptor to attach and infect cells.

The invention provides glycoprotein-immunoglobulin fusion proteins (refereed to herein as "AV fusion protein or AV fusion peptides") containing multiple Siaα3Galβ4GlcNAcβ, Siaα3Galβ3GlcNAcβ, epitopes, that are useful in blocking (i.e., inhibiting) the adhesion interaction between a virus and a cell. The epitopes are terminal, i.e, at the terminus of the glycan. The AV fusion protein inhibits 10%, 20%, 30%, 40%, 50%, 60%, 70%, 80%, 90%, 95%, 98% or 100% of the virus adhesion to a cell. For example, the AV fusion proteins are useful in inhibiting influenza virus, oculotropic virus or Norwalk virus adhesion to cells.

The AV fusion peptide is more efficient on a carbohydrate molar basis in inhibiting viral adhesion as compared to free saccharrides. The AV fusion peptide inhibits 2, 4, 10, 20, 50, 80, 100 or more-fold greater number of virions as compared to an equivalent amount of free saccharrides.

### Fusion Polypeptides

In various aspects the invention provides fusion proteins that include a first polypeptide containing at least a portion of a glycoprotein, *e.g*., a mucin polypeptide or an alpha-globulin polypeptide, operatively linked to a second polypeptide. As used herein, a "fusion protein" or "chimeric protein" includes at least a portion of a glycoprotein polypeptide operatively linked to a non-mucin polypeptide.

A "mucin polypeptide" refers to a polypeptide having a mucin domain. The mucin polypeptide has one, two, three, five, ten, twenty or more mucin domains. The mucin polypeptide is any glycoprotein characterized by an amino acid sequence substituted with O-glycans. For example, a mucin polypeptide has every second or third amino acid being a serine or threonine. The mucin polypeptide is a secreted protein. Alternatively, the mucin polypeptide is a coll surface protein.

Mucin domains are rich in the amino acids threonine, scrine and proline, where the oligosaccharides are linked via N-acetylgalactosamine to the hydroxy amino acids (O-glycans). A mucin domain comprises or alternatively consists of an O-linked glycosylation site. A mucin domain has 1, 2, 3, 5, 10, 20, 50, 100 or more O-linked glycosylation sites. Alternatively, the mucin domain comprises or alternatively consists of an N-linked glycosylation site. A mucin polypeptide has 50%, 60%, 80%, 90%, 95% or 100% of its mass due to the glycans. Mucin polypeptide is any polypeptide encoded for by a MUC gene (*i.e*., MUC1, MUC2, MUC3, etc.) Alternatively, a mucin polypeptide is P-selectin glycoprotein ligand 1 (PSGL-1), CD34, CD43, CD45, CD96, GlyCAM-1, MAdCAM or red blood cell glycophorins. Preferably, the mucin is PSGL-1.

An " alpha-globulin polypeptide" refers to a serum glycoprotein. Alpha-globulins include for example, enzymes produced by the lungs and liver, and haptoglobin, which binds hemoglobin together. An alpha-globulin is an alpha₁ or an alpha₂ globulin. Alpha₁ globulin is predominantly alpha₁antitrypsin, an enzyme produced by the lungs and liver. Alpha₂ globulin, which includes serum haptoglobin, is a protein that binds hemoglobin to prevent its excretion by the kidneys. Other alphaglobulins are produced as a result of inflammation, tissue damage, autoimmune diseases, or certain cancers. Preferably, the alpha-globulin is alpba-1-acid glycoprotein (i.e., orosomucoid.

A "non-mucin polypeptide" refers to a polypeptide of which at least less than 40% of its mass is due to glycans.

Within a AV fusion protein of the invention the mucin polypeptide corresponds to all or a portion of a mucin protein. A AV fusion protein comprises at least a portion of a mucin protein. "At least a portion" is meant that the mucin polypeptide contains at least one mucin domain (*e.g*., an O-linked glycosylation site). The mucin protein comprises the extracellular portion of the polypeptide. For example, the mucin polypeptide comprises the extracellular portion of PSGL-1.

The alpha globulin polypeptide can correspond to all or a portion of a alpha globulin polypeptide. A AV fusion protein comprises at least a portion of an alpha globulin polypeptide "At least a portion" is meant that the alpha globulin polypeptide contains at least one N-linked glycosylation site.

The first polypeptide is glycosylated by one or more glycosyltransferases. The first polypeptide is glycosylated by 2, 3, 5 or more glycosyltransferases. Glycosylation is sequential or consecutive. Alternatively glycosylation is concurrent or random, *i.e*., in no particular order. The first polypeptide is glycosylated by any enzyme capable of adding N-linked or O-linked sialic acid determinants to a protein backbone. For example the first polypeptide is glycosylated by one or more of the following: a core 2 β6-*N*-acetylglucosaminyltransferase, a core 3 β 3-*N-*acetylglucosaminyltransferase, a β4-galactosyltransferase, a β3-galactosyltransferase, an α 3-sialyltransferase, an α6-sialyltransferase, an α2-fucosyltransferase, an α3/4-fucosyltransferase, and/or an α3-*N*-acetylgalactosaminyltransferase. The first polypeptide is more heavily glycosylated than the native (i.e. wild-type) glycoprotein. For example, the first polypeptide has 2, 3, 4, 5, 6, 7, 8, 9, or 10 fold or more glycans than a native glycoprotein. The first polypeptide contains greater that 40%, 50%, 60%, 70%, 80%, 90% or 95% of its mass due to carbohydrate.

Within the fusion protein, the term "operatively linked" is intended to indicate that the first and second polypeptides are chemically linked (most typically via a covalent bond such as a peptide bond) in a manner that allows for O-linked and/or N-linked glycosylation of the first polypeptide. When used to refer to nucleic acids encoding a fusion polypeptide, the term operatively linked means that a nucleic acid encoding the mucin or alpha globulin polypeptide and the non-mucin polypeptide are fused in-frame to each other. The non-mucin polypeptide can be fused to the N-terminus or C-terminus of the mucin or alpha globulin polypeptide.

The AV fusion protein is linked to one or more additional moieties. For example, the AV fusion protein may additionally be linked to a GST fusion protein in which the AV fusion protein sequences are fused to the C-terniinus of the GST (*i.e*., glutathione S-transferase) sequences. Such fusion proteins can facilitate the purification of the AV fusion protein. Alternatively, the AV fusion protein may additionally be linked to a solid support. Various solid supports are known to those skilled in the art. Such compositions can facilitate removal of anti-blood group antibodies. For example, the AV fusion protein is linked to a particle made of, *e.g*., metal compounds, silica, latex, polymeric material; a microtiter plate; nitrocellulose, or nylon or a combination thereof. The AV fusion proteins linked to a solid support are used as an absorber to remove microbes or bacterial toxins from a biological sample, such as gastric tissue, blood or plasma.

The fusion protein includes a heterologous signal sequence (*i.e*., a polypeptide sequence that is not present in a polypeptide encoded by a mucin or a globulin nucleic acid) at its N-terminus. For example, the native mucin or alpba-glycoprotein signal sequence can be removed and replaced with a signal sequence from another protein. In certain host cells (*e.g*., mammalian host cells), expression and/or secretion of polypeplide can be increased through use of a heterologous signal sequence.

A chimeric or fusion protein of the invention can be produced by standard recombinant DNA techniques. For example, DNA fragments coding for the different polypeptide sequences are ligated together in-frame in accordance with conventional techniques, *e.g*., by employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. The fusion gene is synthesized by conventional techniques including automated DNA synthesizers. Alternatively, PCR amplification of gene fragments is carried out using anchor primers that give rise to complementary overhangs between two consecutive gene fragments that can subsequently be annealed and reamplified to generate a chimeric gene sequence (see, for example, Ausubel et al. (eds.) CURRENT PROTOCOLS IN MOLECULAR BIOLOGY, John Wiley & Sons, 1992). Moreover, many expression vectors are commercially available that encode a fusion moiety (*e.g*., an Fc region of an immunoglobulin heavy chain), A mucin or an alpha-globulin encoding nucleic acid can be cloned into such an expression vector such that the fusion moiety is linked in-frame to the immunoglobulin protein.

AV fusion polypeptides may exist as oligomers, such as dimers, timers or pentamers. Preferably, the AV fusion polypeptide is a dimer.

The first polypeptide, and/or nucleic acids encoding the first polypeptide, is constructed using mucin or alpha-globulin encoding sequences are known in the art. Suitable sources for mucin polypeptides and nucleic acids encoding mucin polypeptides include GenBank Accession Nds. NP663625 and NM145650, CAD10625 and AJ417815, XP140694 and XM140694, XP006867 and XM006867 and NP00331777 and NM009151 respectively, and are incorporated herein by reference in their entirety. Suitable sources for alpha-globulin polypeptides and nucleic acids encoding alpha-globulin polypeptides include GenBank Accession Nos. AAH26238 and BC026238; NP000598; and BC012725, AAH12725 and BC012725, and NP44570 and NM053288 respectively, and are incorporated herein by reference in their entirety.

The mucin polypeptide moiety is provided as a variant mucin polypeptide having a mutation in the naturally-occurring mucin sequence (wild type) that results in increased carbohydrate content (relative to the non-mutated sequence). For example, the variant mucin polypeptide comprised additional O-linked glycosylation sites compared to the wild-type mucin. Alternatively, the variant mucin polypeptide comprises an amino acid sequence mutations that results in an increased number of serine, threonine or proline residues as compared to a wild type mucin polypeptide. This increased carbohydrate content can be assessed by determining the protein to carbohydrate ratio of the mucin by methods known to those skilled in the art.

Similarly, the alpha-globulin polypeptide moiety is provided as a variant alpha-globulin polypeptide having a mutation in the naturally-occurring alpha-globulin sequence (wild type) that results in increased carbohydrate content (relative to the non-mutated sequence). For example, the variant alpha-globulin polypeptide comprised additional N-linked glycosylation sites compared to the wild-type alpha-globulin.

Alternatively, the mucin or alpha-globulin polypeptide moiety is provided as a variant mucin or alpha-globulin polypeptide having mutations in the naturally-occurring mucin or alpha-globulin sequence (wild type) that results in a mucin or alpha-globulin sequence more resistant to proteolysis (relative to the non-mutated sequence).

The first polypeptide includes full-length PSGL-1. Alternatively, the first polypeptide comprise less than full-length PSGL-1 polypeptide such as the extracellular portion of PSGL-1. For example the first polypeptides is less than 400 amino acids in length, *e.g*., less than or equal to 300, 250, 150, 100, 50, or 25 amino acids in length.

The first polypeptide includes full-length alpha acid-globulin. Alternatively, the first polypeptide comprises less than full-length alpha acid globulin polypeptides. For example the first polypeptide is less than 200 amino acids in length, *e.g*., less than or equal to 150, 100, 50, or 25 amino acids in length.

The second polypeptide is preferably soluble. In some embodiments, the second polypeptide includes a sequence that facilitates association of the AV fusion polypeptide with a second mucin or alpha globulin polypeptide. The second polypeptide includes at least a region of an immunoglobulin polypeptide. "At least a region" is meant to include any portion of an immunoglobulin molecule, such as the light chain, heavy chain, FC region, Fab region, Fv region or any fragment thereof. Immunoglobulin fusion polypeptide are known in the art and are described in *e.g*., US Patent Nos. 5,516,964; 5,225,538; 5,428,130; 5,514,582; 5,714,147;and 5,455,165.

The second polypeptide comprises a full-length immunoglobulin polypeptide. Alternatively, the second polypeptide comprises less than full-length immunoglobulin polypeptide, *e.g*., a heavy chain, light chain, Fab, Fab₂, Fv, or Fc. Preferably, the second polypeptide includes the heavy chain of an immunoglobulin polypeptide. More preferably the second polypeptide includes the Fc region of an immunoglobulin polypeptide.

The second polypeptide has less effector function than the effector function of a Fc region of a wild-type immunoglobulin heavy chain. Alternatively, the second polypeptide has similar or greater effector function of a Fc region of a wild-type immunoglobulin heavy chain. An Fc effector function includes for example, Fc receptor binding, complement fixation and T cell depleting activity. (*see for example,* US Patent No. 6,136,310) Methods of assaying T cell depleting activity, Fc effector function, and antibody stability are known in the art. In one embodiment the second polypeptide has low or no affinity for the Fc receptor. Alternatively, the second polypeptide has low or no affinity for complement protein C1q.

The polypeptides in question can be produced using to vectors, preferably expression vectors, containing a nucleic acid encoding mucin polypeptides. The vector contains a nucleic acid encoding a mucin or alpha globulin polypeptide operably linked to a nucleic acid encoding an immunoglobulin polypeptide, or derivatives, fragments analogs or homologs thereof. Additionally, the vector comprises a nucleic acid encoding a glycosyltransferase such as an α2-fucosyltransferase. As used herein, the term "vector" refers to a nucleic acid molecule capable of transporting another nucleic acid to which it has been linked. One type of vector is a "plasmid", which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (*e.g*., bacterial vectors having a bacterial origin of replication and episomal mammalian vectors). Other vectors (*e.g*., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors". In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (*e*.*g*., replication defective retroviruses, adenoviruses and adeno-associated viruses), which serve equivalent functions.

The recombinant expression vectors of the invention comprise a nucleic acid of the invention in a form suitable for expression of the nucleic acid in a host cell, which means that the recombinant expression vectors include one or more regulatory sequences, selected on the basis of the host cells to be used for expression, that is operatively-linked to the nucleic acid sequence to be expressed. Within a recombinant expression vector, "operably-linked" is intended to mean that the nucleotide sequence of interest is linked to the regulatory sequence(s) in a manner that allows for expression of the nucleotide sequence (*e.g*., in an *in vitro* transcription/translation system or in a host cell when the vector is introduced into the host cell).

The term "regulatory sequence" is intended to includes promoters, enhancers and other expression control elements (*e.g*., polyadenylation signals). Such regulatory sequences are described, for example, in Goeddel, GENE EXPRESSION TECHNOLOGY: METHODS IN ENZYMOLOGY 185, Academic Press, San Diego, Calif. (1990). Regulatory sequences include those that direct constitutive expression of a nucleotide sequence in many types of host cell and those that direct expression of the nucleotide sequence only in certain host cells (*e.g*., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein (*e.g*., LAV fusion polypeptides, mutant forms of AV fusion polypeptides, etc.).

The recombinant expression vectors of the invention can be designed for expression of AV fusion polypeptides in prokaryotic or eukaryotic cells. For example, AV fusion polypeptides can be expressed in bacterial cells such as *Escherichia coli,* insect cells (using baculovirus expression vectors) yeast cells or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated *in vitro,* for example using T7 promoter regulatory sequences and T7 polymerase.

Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: (*i*) to increase expression of recombinant protein; (*ii*) to increase the solubility of the recombinant protein; and (*iii*) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E*. *coli* expression vectors include pTrc (Amrann et al., (1988) Gene 69:301-315) and pET 11d (Studier et al., GENE EXPRESSION TECHNOLOGY: METHODS IN ENZYMOLOGY 185, Academic Press, San Diego, Calif. (1990) 60-89).

One strategy to maximize recombinant protein expression in *E*. *coli* is to express the protein in a host bacteria with an impaired capacity to proteolytically cleave the recombinant protein. *See*, *e.g*., Gottesman, GENE EXPRESSION TECHNOLOGY: METHODS IN ENZYMOLOGY 185, Academic Press, San Diego, Calif. (1990) 119-128. Another strategy is to alter the nucleic acid sequence of the nucleic acid to be inserted into an expression vector so that the individual codons for each amino acid are those preferentially utilized in *E. coli* (*see, e.g.,* Wada, et al., 1992. Nucl. Acids Res. 20: 2111-2118). Such alteration of nucleic acid sequences of the invention can be carried out by standard DNA synthesis techniques.

The AV fusion polypeptide expression vector is a yeast expression vector. Examples of vectors for expression in yeast *Saccharomyces cerivisae* include pYepSec1 (Baldari, et al., 1987. EMBO J. 6: 229-234), pMFa (Kurjan and Herskowitz, 1982. Cell 30: 933-943), pJRY88 (Schultz et al., 1987. Gene 54: 113-123), pYES2 (Invitrogen Corporation, San Diego, Calif.), and picZ (InVitrogen Corp, San Diego, Calif.).

Alternatively, AV fusion polypeptide can be expressed in insect cells using baculovirus expression vectors. Baculovirus vectors available for expression of proteins in cultured insect cells (*e.g*., SF9 cells) include the pAc series (Smith, et al., 1983. Mol. Cell. Biol. 3: 2156-2165) and the pVL series (Lucklow and Summers, 1989. Virology 170: 31-39).

A nucleic acid of the invention is expressed in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are often provided by viral regulatory elements. For example, commonly used promoters are derived from polyoma, adenovirus 2, cytomegalovirus, and simian virus 40. For other suitable expression systems for both prokaryotic and eukaryotic cells see, *e.g*., Chapters 16 and 17 of Sambrook, et al., MOLECULAR CLONING: A LABORATORY MANUAL. 2nd ed., Cold Spring Harbor Laboratory, Cold Spring Harbor Laboratory Press, Cold Spring Harbor, N.Y., 1989.

Another aspect of the invention pertains to host cells into which a recombinant expression vector of the invention has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell. For example, AV fusion polypeptides can be expressed in bacterial cells such as *E. coli,* insect cells, yeast or mammalian cells (such as human, Chinese hamster ovary cells (CHO) or COS cells). Other suitable host cells are known to those skilled in the art.

Vector DNA can be introduced into prokaryotic or eukaryotic cells via conventional transformation or transfection techniques. As used herein, the terms "transformation" and "transfection" are intended to refer to a-variety of art-recognized techniques for introducing foreign nucleic acid (*e.g*., DNA) into a host cell, including calcium phosphate or calcium chloride co-precipitation, DEAE-dextran-mediated transfection, lipofection, or electroporation. Suitable methods for transforming or transfecting host cells can be found in Sambrook, et al. (MOLECULAR CLONING: A LABORATORY MANUAL. 2nd ed., Cold Spring Harbor Laboratory, Cold Spring Harbor Laboratory Press, Cold Spring Harbor, N.Y., 1989), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (*e.g*., resistance to antibiotics) is generally introduced into the host cells along with the gene of interest. Various selectable markers include those that confer resistance to drugs, such as G418, hygromycin and methotrexate. Nucleic acid encoding a selectable marker can be introduced into a host cell on the same vector as that encoding the fusion polypeptides or can be introduced on a separate vector. Cells stably transfected with the introduced nucleic acid can be identified by drug selection (*e.g*., cells that have incorporated the selectable marker gene will survive, while the other cells die).

A host cell of the invention, such as a prokaryotic or eukaryotic host cell in culture, can be used to produce (*i.e*., express) AV fusion polypeptides. Accordingly, the invention further provides methods for producing AV fusion polypeptides using the host cells of the invention. In one embodiment, the method comprises culturing the host cell of invention (into which a recombinant expression vector encoding AV fusion polypeptides has been introduced) in a suitable medium such that AV fusion polypeptides is produced. In another embodiment, the method further comprises isolating AV polypeptide from the medium or the host cell.

The AV fusion polypeptides may be isolated and purified in accordance with conventional conditions, such as extraction, precipitation, chromatography, affinity chromatography, electrophoresis or the like. For example, the immunoglobulin fusion proteins may be purified by passing a solution through a column which contains immobilized protein A or protein G which selectively binds the Fc portion of the fusion protein. See, for example, Reis, K. J., et al., J. Immunol. 132:3098-3102 (1984); PCT Application, Publication No. WO87/00329. The fusion polypeptide may the be eluted by treatment with a chaotropic salt or by elution with aqueous acetic acid (1 M).

Alternatively, an AV fusion polypeptides according to the invention can be chemically synthesized using methods known in the art. Chemical synthesis of polypeptides is described in, *e.g*., A variety of protein synthesis methods are common in the art, including synthesis using a peptide synthesizer. See, *e.g.,* Peptide Chemistry, A Practical Textbook, Bodasnsky, Ed. Springer-Verlag, 1988: Merrifield, Science 232: 241-247 (1986); Barany, et at, Intl. J. Peptide Protein Res. 30: 705-739 (1987); Kent, Ann. Rev. Biochem. 57:957-989 (1988), and Kaiser, et al, Science 243: 187-198 (1989). The polypeptides are purified so that they are substantially free of chemical precursors or other chemicals using standard peptide purification techniques. The language "substantially free of chemical precursors or other chemicals" includes preparations of peptide in which the peptide is separated from chemical precursors or other chemicals that are involved in the synthesis of the peptide. In one embodiment, the language "substantially free of chemical precursors or other chemicals" includes preparations of peptide having less than about 30% (by dry weight) of chemical precursors or non-peptide chemicals, more preferably less than about 20% chemical precursors or non-peptide chemicals, still more preferably less than about 10% chemical precursors or non-peptide chemicals, and most preferably less than about 5% chemical precursors or non-peptide chemicals.

Chemical synthesis of polypeptides facilitates the incorporation of modified or unnatural amino acids, including D-amino acids and other small organic molecules. Replacement of one or more L-amino acids in a peptide with the corresponding D-amino acid isoforms can be used to increase the resistance of peptides to enzymatic hydrolysis, and to enhance one or more properties of biologically active peptides, *i.e*., receptor binding, functional potency or duration of action. See, *e.g.,* Doherty, et al., 1993. J. Med. Chem. 36: 2585-2594; Kirby, et al., 1993. J. Med. Chem, 36:3802-3808; Morita, et al., 1994. FEBS Lett. 353: 84-88; Wang, et al., 1993. Int. J. Pept. Protein Res. 42: 392-399; Fauchere and Thiunieau, 1992. Adv. Drug Res. 23: 127-159.

Introduction of covalent cross-links into a peptide sequence can conformation ally and topographically constrain the polypeptide backbone. This strategy can be used to develop peptide analogs of the fusion polypeptides with increased potency, selectivity and stability. Because the conformational entropy of a cyclic peptide is lower than its linear counterpart, adoption of a specific conformation may occur with a smaller decrease in entropy for a cyclic analog than for an acyclic analog, thereby making the free energy for binding more favorable. Macrocyclization is often accomplished by forming an amide bond between the peptide N- and C-termini, between a side chain and the N- or C-teminus [*e.g.,* with K₃Fe(CN)₆ at pH 8.5] (Samson et al., Endocrinology, 137: 5182-5185 (1996)), or between two amino acid side chains. See, *e.g*., DeGrado, Adv Protein Chem, 39: 51-124 (1988). Disulfide bridges are also introduced into linear sequences to reduce their flexibility. See, *e.g*., Rose, et al., Adv Protein Chem, 37: 1-109 (1985); Mosberg et al., Biochem Biophys Res Commun, 106: 505-512 (1982). Furthermore, the replacement of cysteine residues with penicillamine (Pen, 3-mercapto-(D) valine) has been used to increase the selectivity of some opioid-receptor interactions. Lipkowski and Carr, Peptides: Synthesis, Structures, and Applications, Gutte, ed., Academic Press pp. 287-320(1995).

### Methods of Decreasing Viral Attachment

Viral attachment to a cell is inhibited (e.g. decreased) by contacting a virus with the AV fusion peptide of the invention. The virus is of example, an avian Influenza A virus.

Inhibition of attachment is characterized by a decrease in viral entry and replication. Viruses are directly contacted with the AV peptide. Alternatively, the AV peptide is administered to a subject systemically. AV peptides are administered in an amount sufficient to decrease (e.g., inhibit) viral attachment. Attachment is measured using standard adhesion assays known in the art, e.g. by measuring viral attachment to cells using radioactively, or by other means, labeled viruses, by detecting attached viruses using anti-viral antibodies, or by measuring produced viral products following viral replication.

The methods are useful to alleviate the symptoms of a variety of viral infections or a disease associated with a viral infection. The viral infection is for example, influenza virus or a calici virus infection. Diseases associated with viral infection include for example, pneumonia and gastroenteritis.

The methods described herein lead to a reduction in the severity or the alleviation of one or more symptoms of a viral infection or disorder such as those described herein. Viral infection or disorders associated with a viral infection are diagnosed and or monitored, typically by a physician using standard methodologies.

The subject is *e.g*., any mammal, *e.g*., a human, a primate, mouse, rat, dog, cat, cow, horse, pig. The treatment is administered prior to microbial infection or diagnosis of the disorder. Alternatively, treatment is administered after a subject has an infection.

Efficaciousness of treatment is determined in association with any known method for diagnosing or treating the particular microbial infection or disorder associated with a viral infection. Alleviation of one or more symptoms of the viral infection or disorder indicates that the compound confers a clinical benefit.

### Exemplary Viruses

*Influenza virus*: Influenza A viruses are highly, but not completely, species- and receptor-specific. Avian influenza A viruses that use α2,3-linked sialic acid as receptor do not easily infect man and human influenza A viruses that use α2,6-linked sialic acid do not easily infect aquatic birds. The human respiratory tract is abundant in α2,6-linked sialic acid, and recently evidence was presented that non-ciliated tracheal cells are the primary target for human influenza virus. In contrast to non-ciliated cells of the trachea, its ciliated cells contain α 2,3-linked sialic acid and they are able to support replication of some avian influenza variants. Influenza viruses can also exhibit organ-specificity. For example, during the avian H7N7 Dutch outbreak in 2003, the major manifestation of the infection in human beings was ocular rather than respiratory. The virus was suggested to be transmitted from the primary cases to more than 50% of their household contacts. Thus, both the eye and the respiratory tract may serve as a colonization entrance in humans for avian influenza A viruses.

*Oculotropic viruses:* Adenoviridae is a large family with approximately 50 genotypes that causes mainly respiratory or gastrointestinal symptoms. Ad8, Ad19 and Ad37 infect the eye, the most important disease being epidemic keratoconjunctivitis. These adenoviridae exhibit tropism for the eye by binding α2,3-linked sialic acid, which is the most frequent type of sialic acid linkage in corneal and conjunctival cells. Interestingly, mucins of the tear fluid carry glycans terminating with α2,6-linked sialic acid, and is consequently inefficient in terms of binding and blocking invading oculotropic adenoviruses. Similarly, enterovirus 70 (EV70) also uses α2,3-linked sialic acid as its receptor. It causes a somewhat less severe, but even more contagious eye disease, known as acute hemorrhagic conjunctivitis.

*Norwalk virus:* Only a few human viruses use neutral glycoepitopes as receptors and human parvovirus B19 and some members of the Norovirus genus are the best known examples. Noroviruses cause severe outbreaks of diarrhea and vomiting in the general population as wall as among patients and staff members of hospitals and other ward institutions. Histo-blood group ABH antigens are likely receptors for Noroviruses, and a functional FUT2 (*Secretor*) gene is a prerequisite for an individual to be susceptible to Norovirus infection. It has also been shown that the blood group H antigen needs to be carried by specific core saccharide chains, namely types 1 (Galβ1,3GlcNAc) and 3 (Galβ1,3GalNAca), in order to act as receptors for many Noroviruses. In addition, Norovirus genogroup I (*e.g*. Norwalk virus) and genogroup II (*e.g.* Snow Mountain virus) differ in receptor preference also regarding the ability to bind ABO histo-blood group antigens. Three binding patterns have been described sofar: Norwalk virus (genogroup I) binds A/O, strain MOH (genotype II) binds A/B, and strain VA387 binds A/B/O. Approximately 20 % of Caucasians and Africans are non-secretors, *i.e*. carry a defect FUT2 gene, and are naturally resistant to most Norovirus strains. In addition to these binding specificities it has recently been shown that some Norovirus strains can accept additional monosaccharide substitutions of above mentioned carbohydrate epitopes. For instance, apart from blood group H and A, related structures such as A Lewis b and A Lewis y are bound. Also, although core saccharide chains 1 and 3 seem to be preferred, type 2 chain based structures, *e.g*. A2 and above mentioned A Lewis y, can also be recognized by some strains.

### Pharmaceutical Compositions Including AV Fusion Polypeptides or Nucleic Acids Encoding Same

The AV fusion proteins, or nucleic acid molecules encoding these fusion proteins, (also referred to herein as "Therapeutics" or "active compounds") of the invention, and derivatives, fragments, analogs and homologs thereof, can be incorporated into pharmaceutical compositions suitable for administration. Such compositions typically comprise the nucleic acid molecule, protein, or antibody and a pharmaceutically acceptable carrier. As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field, which is incorporated herein by reference. Preferred examples of such carriers or diluents include, but are not limited to, water, saline, finger's solutions, dextrose solution, and 5% human serum albumin. Liposomes and non-aqueous vehicles such as fixed oils may also be used. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The active agents disclosed herein can also be formulated as liposomes. Liposomes are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter.

A pharmaceutical composition of the invention is formulated to be compatible with its intended route of administration. Examples of routes of administration include parenteral, *e.g*., intravenous, intradermal, subcutaneous, oral (*e.g*., inhalation), transdermal (*i.e*., topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid (EDTA); buffers such as acetates, citrates or phosphates, and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL^{™} (BASF, Parsippany, N.J.) or phosphate buffered saline (PBS). In all cases, the composition must be sterile and should be fluid to the extent that easy syringeability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as manitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (*e.g*., an AV fusion protein) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from pressured container or dispenser which contains a suitable propellant, *e.g*., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (*e.g*., with conventional suppository bases such as cocoa butter and other glycerides) or retention enemas for rectal delivery.

The active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Patent No. 4,522,811.

Oral or parenteral compositions are formulated in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration (*see*, *e.g*., U.S. Patent No. 5,328,470) or by stereotactic injection (*see*, *e.g*., Chen, et al., 1994. Proc. Natl. Acad. Sci. USA 91: 3054-3057). The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, *e.g.,* retroviral vectors, the pharmaceutical preparation can include one or more cells that produce the gene delivery system.

Sustained-release preparations can be prepared, if desired. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers ofL-glutamic acid and γ ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

The pharmaceutical compositions can be included in a container, pack, or dispenser together with instructions for administration.

The invention will be further illustrated in the following non-lirniting examples.

### EXAMPLE 1: ENGINEERING STABLE CELL LINES SECRETING I_{G}^{G} FC FUSIONS OF P-SELECTIN GLYCOPROTEIN LIGAND-1 AND α₁-ACID GLYCOPROTEIN CARRYING SIAα3GALβ4GLCNACβ GLYCANS

The PSGL-1/mIgG_{2b} or AGP/mIgG_{2b} expression plasmids will be stably transfected alone into COS or 293 cells having endogenous core 2 α6GlcNAc transferase (T) activity, or together with the core 2 β6GlcNAc-T1 into CHO-K1 cells. All of these cell lines have endogenous β1,4galactosyltransferase activity that will make the type 2 chain (Ga1β1,4GlcNAc), and α2,3-sialyltransferase activity that will carry out the final sialylation step during the biosynthesis of the desired epitope, Siaα3Galβ4GlcNAcβ. Stable clones are selected based on resistance to different selection drugs, *e.g*. puromycin and zeocin.

### EXAMPLE 2: ENGINEERING STABLE CELL LINES SECRETING IGG FC FUSIONS OF P-SELECTIN GLYCOPROTEIN LIGAND-1 AND α₁-ACED GLYCOPROTEIN CARRYING SIAα6GALβ4GLCNAβ GLYCANS

Cell lines made as described above, will be stably transfected with α2,6-sialyltransferase cDNAs (ST6GalT I or II) in order to divert the sialylation towards α2,6-linked sialic acid. In order to reduce α2,3-sialylation it may become necessary to down-regulate α 2,3-sialyltransferase expression by the use of siRNAs cleaving α2,3-sialyltransferase mRNAs.

### EXAMPLE 3: ENGINEERING STABLE CELL LINES SECRETING IGG FC FUSIONS OF P-SELECTIN GLYCOPROTEIN LIGAND-1 AND α₁-ACID GLYCOPROTEIN CARRYING FUCα2GALβ3GALNACβ-SER/THR OR FUCα2GALβ3GLCNACβ-R GLYCANS

CHO-K1 cells will be stably transfected with the PSGL-1/mIgG_{2b} or AGP/mIgG_{2b} expression plasmids and the FUT2 gene in order to obtain the Fucα2Galβ3GalNAcβ-Ser/Thr determinant on the fusion proteins, and with core 3 β3GlcNAc-T6, β3Gal-TV and FUT2 in order to get the Fucα2Galβ3GleNAcβ-R determinant. In order to reduce α2,3/6-sialylation it may become necessary to down-regulate α2,3/6-sialyltransferase expression by the use of siRNAs cleaving α2,3/6-sialyltransferase mRNAs.

### EXAMPLE 4: INHIBITING VIRAL ADHESION AND INFECTION IN VITRO

Relevant viruses and target cells will be used to assess the inhibitory capacity of the above described fusion proteins with regards to preventing viral attachment and replication in susceptible host cells.

### EXAMPLE 5: ROUTES OF ADMINISTRATION

We anticipate to administer recombinant PSGL-1/ or AGP/mIgG_{2b} carrying Siaα3Galβ4GlcNAcβ locally in the eye in order to treat or prevent conjunctivitis caused by oculotropic viruses such as avian influenza, adenovirus 37 and enterovirus 70. The Siaα6Galβ4GlcNAcβ-substituted recombinant fusion proteins will be inhaled as a powder or an aerosol in order to treat or prevent human influenza A virus infection of the respiratory tract. Norovirus infection will be treated or prevented by oral ingestion or inhalation of recombinant IgG fusion proteins of PSGL-1, or a similar mucin-type protein, or AGP carrying blood group H epitopes (Fucα2Galβl-R) based on type 3 or type 1.

## Claims

1. A fusion polypeptide for use in a method for decreasing adhesion of a virus to a cell by contacting the virus with said fusion polypeptide, wherein the fusion polypeptide comprises a first polypeptide operably linked to a second polypeptide wherein the first polypeptide carries at least one glycan selected from the group consisting of:
a) a Siaα3Galβ4GlcNAcβ glycan,
b) a Siaα3Galβ3GlcNAcβ glycan,
and the second polypeptide comprises at least a region of an immunoglobulin polypeptide,
and wherein the first polypeptide is a mucin polypeptide which includes an extracellular portion of a P-selectin glycoprotein ligand-1 or wherein the first polypeptide comprises at least a region of an alpha glycoprotein polypeptide.

2. A fusion polypeptide for use in preventing viral infection or alleviating a symptom of a virus infection in a subject in need thereof, wherein the fusion polypeptide comprises a first polypeptide operably linked to a second polypeptide wherein the first polypeptide carries at least one glycan selected from the group consisting of:
a) a Siaα3Galβ4GlcNAcβ glycan,
b) a Siaα3Galβ3GlcNAcβ glycan,
and the second polypeptide comprises at least a region of an immunoglobulin polypeptide,
and wherein the first polypeptide is a mucin polypeptide which includes an extracellular portion of a P-selectin glycoprotein ligand-1 or wherein the first polypeptide comprises at least a region of an alpha glycoprotein polypeptide.

3. The fusion polypeptide for use according to any one of the preceding claims, wherein said glycan is terminal.

4. The fusion polypeptide for use of any one of claims 1 or 2, wherein said glycan is multivalent.

5. The fusion polypeptide for use of any one of claims 1 or 2, wherein the second polypeptide comprises a region of a heavy chain immunoglobulin polypeptide.

6. The fusion polypeptide for use of any one of claims 1 or 2, wherein said second polypeptide comprises an Fc region of an immunoglobulin heavy chain.

7. The fusion polypeptide for use according to any one of the preceding claims, wherein the polypeptide prevents adhesion of a virus that causes respiratory infection.

8. The fusion polypeptide for use of claims 1 or 2, wherein the virus is an oculotropic virus, a human influenza virus, an avian influenza virus, a recombination of a human or an avian influenza virus.

9. The fusion polypeptide for use of claim 8, wherein the oculotropic virus is an adenovirus 37, adenovirus 19a, adenovirus 8, an enterovirus 70 or an avian influenza virus.

## Patentansprüche

1. Fusionspolypeptid zur Verwendung in einem Verfahren zum Herabsetzen der Anheftung eines Virus an eine Zelle durch In-Kontakt-Bringen des Virus mit dem Fusionspolypeptid, wobei das Fusionspolypeptid ein erstes Polypeptid umfasst, das funktionsfähig mit einem zweiten Polypeptid verknüpft ist, wobei das erste Polypeptid wenigstens ein Glycan trägt, das ausgewählt ist aus der Gruppe bestehend aus:
a) einem Siaα3Galβ4GlcNAcβ-Glycan,
b) einem Siaα3Galβ3GlcNAcβ-Glycan,
und das zweite Polypeptid wenigstens eine Region eines Immunglobulinpolypeptids umfasst,
und wobei das erste Polypeptid ein Mucin-Polypeptid ist, welches einen extrazellulären Teil eines P-Selectin-Glykoprotein-Liganden-1 enthält, oder wobei das erste Polypeptid wenigstens eine Region eines alpha-Glykoproteinpolypeptids umfasst.

2. Fusionspolypeptid zur Verwendung bei der Prävention von Virusinfektionen oder zur Linderung eines Symptoms einer Virusinfektion in einem betroffenen Subjekt, wobei das Fusionspolypeptid ein erstes Polypeptid umfasst, das funktionsfähig mit einem zweiten Polypeptid verknüpft ist, wobei das erste Polypeptid wenigstens ein Glycan trägt, das ausgewählt ist aus der Gruppe bestehend aus:
a) einem Siaα3Galβ4GlcNAcβ-Glycan,
b) einem Siaα3Galβ3GlcNAcβ-Glycan,
und das zweite Polypeptid wenigstens eine Region eines Immunglobulinpolypeptids umfasst,
und wobei das erste Polypeptid ein Mucin-Polypeptid ist, welches einen extrazellulären Teil eines P-Selectin-Glykoprotein-Liganden-1 enthält, oder wobei das erste Polypeptid wenigstens eine Region eines alpha-Glykoproteinpolypeptids umfasst.

3. Fusionspolypeptid zur Verwendung nach einem der vorhergehenden Ansprüche, wobei das Glycan endständig ist.

4. Fusionspolypeptid zur Verwendung nach einem der Ansprüche 1 oder 2, wobei das Glycan multivalent ist.

5. Fusionspolypeptid zur Verwendung nach einem der Ansprüche 1 oder 2, wobei das zweite Polypeptid eine Region eines Polypeptids einer schweren Immunglobulinkette umfasst.

6. Fusionspolypeptid zur Verwendung nach einem der Ansprüche 1 oder 2, wobei das zweite Polypeptid eine Fc-Region einer schweren Immunglobulinkette umfasst.

7. Fusionspolypeptid zur Verwendung nach einem der vorhergehenden Ansprüche, wobei das Polypeptid die Anheftung eines Virus verhindert, das Atemwegserkrankungen verursacht.

8. Fusionspolypeptid zur Verwendung nach einem der Ansprüche 1 oder 2, wobei das Virus ein okulotropes Virus, ein Humaninfluenzavirus, ein Vogelinfluenzavirus oder eine Rekombination eines Human- oder einem Vogelinfluenzavirus ist.

9. Fusionspolypeptid zur Verwendung nach Anspruch 8, wobei das okulotrope Virus ein Adenovirus 37, ein Adenovirus 19a, ein Adenovirus 8, ein Enterovirus 70 oder ein Vogelinfluenzavirus ist.

## Revendications

1. Polypeptide de fusion pour utilisation dans un procédé pour réduire l'adhérence d'un virus à une cellule par mise en contact du virus avec ledit polypeptide de fusion, lequel polypeptide de fusion comprend un premier polypeptide lié de manière fonctionnelle à un deuxième polypeptide, dans lequel le premier polypeptide porte au moins un glycane choisi dans l'ensemble constitué par :
a) un glycane Siaα3Galβ4GlcNAcβ,
b) un glycane Siaα3Galβ3GlcNAcβ,
et le deuxième polypeptide comprend au moins une région d'un polypeptide d'immunoglobuline,
et dans lequel le premier polypeptide est un polypeptide de mucine qui comprend une partie extracellulaire d'un ligand-1 de glycoprotéine P-sélectine ou dans lequel le premier polypeptide comprend au moins une région d'un polypeptide d'α-glycoprotéine.

2. Polypeptide de fusion pour utilisation dans la prévention d'une infection virale ou le soulagement d'un symptôme d'une infection virale chez un sujet en ayant besoin, lequel polypeptide de fusion comprend un premier polypeptide lié de manière fonctionnelle à un deuxième polypeptide, dans lequel le premier polypeptide porte au moins un glycane choisi dans l'ensemble constitué par :
a) un glycane Siaα3Galβ4GlcNAcβ,
b) un glycane Siaα3Galβ3GlcNAcβ,
et le deuxième polypeptide comprend au moins une région d'un polypeptide d'immunoglobuline,
et dans lequel le premier polypeptide est un polypeptide de mucine qui comprend une partie extracellulaire d'un ligand-1 de glycoprotéine P-sélectine ou dans lequel le premier polypeptide comprend au moins une région d'un polypeptide d'α-glycoprotéine.

3. Polypeptide de fusion pour utilisation selon l'une quelconque des revendications précédentes, dans lequel ledit glycane est terminal.

4. Polypeptide de fusion pour utilisation selon l'une quelconque des revendications 1 et 2, dans lequel ledit glycane est multivalent.

5. Polypeptide de fusion pour utilisation selon l'une quelconque des revendications 1 et 2, dans lequel le deuxième polypeptide comprend une région d'un polypeptide d'immunoglobuline à chaîne lourde.

6. Polypeptide de fusion pour utilisation selon l'une quelconque des revendications 1 et 2, dans lequel le deuxième polypeptide comprend une région Fc d'une chaîne lourde d'immunoglobuline.

7. Polypeptide de fusion pour utilisation selon l'une quelconque des revendications précédentes, lequel polypeptide empêche l'adhérence d'un virus qui provoque une infection respiratoire.

8. Polypeptide de fusion pour utilisation selon la revendication 1 ou 2, dans lequel le virus est un virus oculotrope, un virus grippal humain, un virus grippal aviaire, une recombinaison d'un virus grippal humain ou aviaire.

9. Polypeptide de fusion pour utilisation selon la revendication 8, dans lequel le virus oculotrope est un adénovirus 37, adénovirus 19a, adénovirus 8, un entérovirus 70 ou un virus grippal aviaire.
